(19) Europäisches Patentamt
European Patent Office
Office européen des brevets

(11) Veröffentlichungsnummer: **0 177 455 A2**

(12) # EUROPÄISCHE PATENTANMELDUNG

(21) Anmeldenummer: 85810451.6

(22) Anmeldetag: 30.09.85

(51) Int. Cl.⁴: C 07 D 213/64
A 01 N 47/34

(30) Priorität: 05.10.84 CH 4798/84

(43) Veröffentlichungstag der Anmeldung: 09.04.86 Patentblatt 86/15

(84) Benannte Vertragsstaaten: AT BE CH DE FR IT LI NL

(71) Anmelder: CIBA-GEIGY AG
Klybeckstrasse 141
CH-4002 Basel(CH)

(72) Erfinder: Drabek, Jozef, Dr.
Benkenstrasse 12
CH-4104 Oberwil(CH)

(72) Erfinder: Böger, Manfred
Wilhelm Glockstrasse 14
D-7858 Weil am Rhein 5(DE)

(72) Erfinder: Steiner, Eginhard, Dr.
Obere Hofackerstrasse 3
CH-4414 Füllinsdorf(CH)

(54) **N-Benzoylphenylharnstoffe.**

(57) Neue substituierte N-Benzoyl-N'-pyridyloxyphenylharnstoffe der Formel

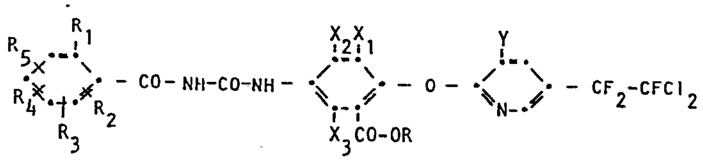

(I)

worin

$X_1$, $X_2$ und $X_3$ unabhängig voneinander Wasserstoff, Halogen oder Methyl;

Y Wasserstoff oder Halogen;

R Wasserstoff, $C_1$-$C_5$-Alkyl, $C_3$-$C_5$-Alkenyl oder $C_3$-$C_5$-Alkinyl; und

$R_1$, $R_2$, $R_3$, $R_4$ und $R_5$ unabhängig voneinander Wasserstoff, Halogen, $C_1$-$C_4$-Alkyl, $C_1$-$C_4$-Alkoxy oder $C_1$-$C_4$-Alkylthio bedeuten; Verfahren und Zwischenprodukte zur Herstellung dieser Verbindungen sowie diese enthaltende Mittel zur Bekämpfung von Insekten und Vertretern der Ordnung Akarina. Die neuen Verbindungen zeichnen sich durch larvizide Wirkung, speziell gegen pflanzenschädigende Insekten, aus.

EP 0 177 455 A2

Croydon Printing Company Ltd.

CIBA-GEIGY AG
Basel (Schweiz)

5-15104/+/ZFO

## N-Benzoylphenylharnstoffe

Die vorliegende Erfindung betrifft neuartige substituierte N-Benzoyl-N'-pyridyloxyphenylharnstoffe, Verfahren und Zwischenprodukte zu ihrer Herstellung sowie ihre Verwendung in der Schädlingsbekämpfung.

Diese erfindungsgemässen Benzoylphenylharnstoff-Derivate haben die Formel I

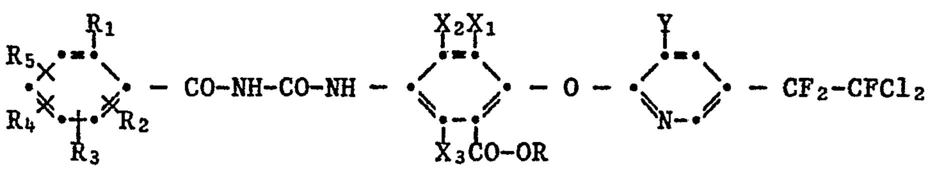 (I),

worin

$X_1$, $X_2$ und $X_3$ unabhängig voneinander Wasserstoff, Halogen oder Methyl;
Y Wasserstoff oder Halogen;
R Wasserstoff, $C_1$-$C_5$-Alkyl, $C_3$-$C_5$-Alkenyl oder $C_3$-$C_5$-Alkinyl; und
$R_1$, $R_2$, $R_3$, $R_4$ und $R_5$ unabhängig voneinander Wasserstoff, Halogen, $C_1$-$C_4$-Alkyl, $C_1$-$C_4$-Alkoxy oder $C_1$-$C_4$-Alkylthio
bedeuten.

Unter dem Begriff Alkyl selbst oder als Bestandteil eines anderen Substituenten sind je nach Zahl der angegebenen Kohlenstoffatome im Rahmen der vorliegenden Erfindung beispielsweise folgende Gruppen zu verstehen: Methyl, Ethyl, Propyl, Butyl, sowie ihre Isomeren, wie z.B. Isopropyl, Isobutyl, tert.-Butyl, sek.-Butyl, Isopentyl usw. Alkenyl steht z.B. für Propenyl-(1), Allyl, Butenyl-(1), Butenyl-(2)

oder Butenyl-(3) und Alkinyl z.B. für Propargyl, Butinyl-(2) oder Pentinyl-(3). Unter Halogen im Rahmen der vorliegenden Erfindung ist vorzugsweise F, Cl und Br zu verstehen, insbesondere F und Cl.

Wegen ihrer Wirkung als Schädlingsbekämpfungsmittel bevorzugt sind Verbindungen der Formel I, die dadurch gekennzeichnet sind, dass
$X_1$, $X_2$ und $X_3$ unabhängig voneinander Wasserstoff, Fluor, Chlor oder Methyl;
Y Wasserstoff, Fluor oder Chlor;
R Wasserstoff, $C_1$-$C_4$-Alkyl, Allyl oder Propargyl; und
$R_1$, $R_2$, $R_3$, $R_4$ und $R_5$ unabhänig voneinander Wasserstoff, Fluor, Chlor, Methyl, Aethyl, Methoxy, Aethoxy oder Methylthio
bedeuten.

Wertvoll sind aufgrund ihrer biologischen Wirksamkeit ferner solche Verbindungen der Formel I, worin
$X_1$ Wasserstoff, Fluor, Chlor oder Methyl;
$X_2$ und $X_3$ Wasserstoff;
Y Wasserstoff oder Chlor;
R Wasserstoff oder $C_1$-$C_4$-Alkyl;
$R_1$ Fluor, Chlor, Methyl, Aethyl, Methoxy, Aethoxy oder Methylthio;
$R_2$ Wasserstoff, 6-Fluor, 6-Chlor, 6-Methyl oder 6-Aethyl; und
$R_3$, $R_4$ und $R_5$ Wasserstoff
bedeuten.

Hervorzuheben sind insbesondere Verbindungen der Formel I, worin
$X_1$ Wasserstoff oder Chlor;
$X_2$ und $X_3$ Wasserstoff;
Y Wasserstoff oder Chlor;
R Wasserstoff oder $C_1$-$C_4$-Alkyl;
$R_1$ Fluor, Chlor oder Methoxy;
$R_2$ Wasserstoff, 6-Fluor oder 6-Chlor; und
$R_3$, $R_4$ und $R_5$ Wasserstoff
bedeuten.

Die Verbindungen der Formel I können nach an sich bekannten Verfahren hergestellt werden (vergl. Europäische Patentschrift Nr. 0.056.124). Man kann z.B. eine Verbindung der Formel I erhalten, indem man

a) eine Verbindung der Formel II

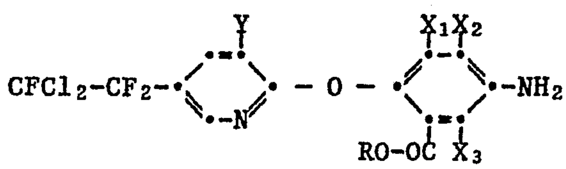

(II)

mit einer Verbindung der Formel III

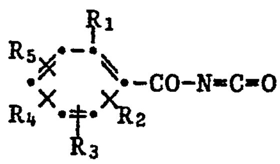

(III),

b) eine Verbindung der Formel IV

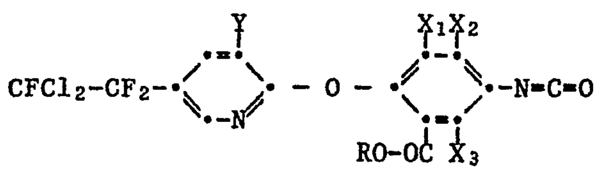

(IV)

mit einer Verbindung der Formel V

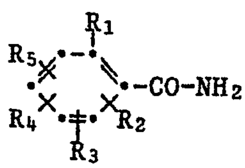

(V) oder

c) eine Verbindung der Formel II mit einer Verbindung der Formel VI

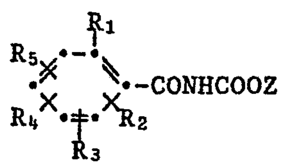

(VI)

umsetzt, wobei in den Formeln II bis VI die Reste $X_1$ bis $X_3$, Y, R und $R_1$ bis $R_5$ die vorstehend angegebenen Bedeutungen haben, und Z eine gegebenenfalls mit Halogen, vorzugsweise Chlor, substituierte $C_1$-$C_8$-Alkylgruppe bedeutet.

Die erwähnten Verfahren a), b) und c) können vorzugsweise unter normalem Druck und in Gegenwart eines organischen Lösungs- oder Verdünnungsmittels durchgeführt werden. Als Lösungs- oder Verdünnungsmittel eignen sich z.B. Aether und ätherartige Verbindungen, wie Diäthyläther, Dipropyläther, Dibutyläther, Dioxan, Dimethoxyäthan und Tetrahydrofuran; N,N-dialkylierte Carbonsäureamide; aliphatische, aromatische sowie halogenierte Kohlenwasserstoffe, insbesondere Benzol, Toluol, Xylol, Chloroform, Methylenchlorid, Tetrachlorkohlenstoff und Chlorbenzol; Nitrile, wie Acetonitril oder Propionitril; Dimethylsulfoxid sowie Ketone, z.B. Aceton, Methyläthylketon, Methylisopropylketon und Methylisobutylketon. Verfahren a) wird im allgemeinen bei einer Temperatur von -10 bis 200°C, vorzugsweise zwischen 0 und 100°C, gegebenenfalls in Gegenwart einer organischen Base, z.B. Triäthylamin, durchgeführt. Die Durchführung von Verfahren b) erfolgt bei einer Temperatur von 0 bis 150°C, vorzugsweise beim Siedepunkt des verwendeten Lösungsmittels, und gegebenenfalls in Gegenwart einer organischen Base, wie Pyridin, und/oder unter Zusatz eines Alkali- oder Erdalkalimetalls, vorzugsweise Natrium. Für das Verfahren c), d.h. für die Umsetzung der Urethane der Formel VI mit einem Pyridyloxyanilin der Formel II, werden Temperaturen zwischen etwa 60°C und dem Siedepunkt des jeweiligen Reaktionsgemisches bevorzugt, wobei als Lösungsmittel insbesondere aromatische Kohlenwasserstoffe, wie Toluol, Xylole, Chlorbenzol usw., verwendet werden.

Die Ausgangsstoffe der Formeln III, V und VI sind bekannt und können analog bekannten Verfahren hergestellt werden. Bei den Ausgangsstoffen der Formeln II handelt es sich um neuartige Verbindungen, die ebenfalls einen Gegenstand der vorliegenden Erfindung bilden.

Die Verbindungen der Formel II können in an sich bekannter Weise dadurch hergestellt werden, dass man entsprechende 5-Aminosalicylsäurederivate der Formel VII

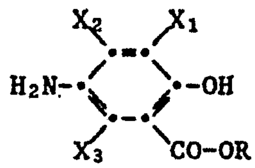

(VII)

mit einem Pyridinderivat der Formel VIII

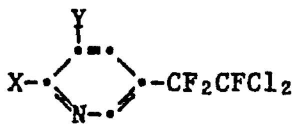

(VIII)

umsetzt.

In den obigen Formeln VII und VIII entsprechen die Reste $X_1$, $X_2$, $X_3$, R und Y den vorstehend unter Formel I angegebenen Definitionen und X steht für F oder Cl.

Substituierte N-Benzoyl-N'-phenylharnstoffe mit insektizider Wirkung, die am Phenylring einen Alkoxycarbonyl-Substituenten aufweisen, sind bereits aus der europäischen Patentschrift Nr. 0.056.124 bekannt. Weiterhin sind aus der deutschen Offenlegungsschrift 3 033 512 insektizid wirksame N-Benzoyl-N'-4-(3-chlor-5-trifluormethyl-pyridyl-2-oxy)-phenylharnstoffe bekannt; entsprechende insektizide N-Benzoyl-N'-phenylharnstoffe mit einem Carboxyl- oder Alkoxycarbonyl-Substituenten in 3-Stellung am Phenylring sind Gegenstand der japanischen Patentveröffentlichungen Nr. 5-7099.569, 5-7109.768, 5-7144.258, 5-7144.259, 5-7145.861 und 5-8039.657. Die erfindungsgemässen Verbindungen der Formel I unterscheiden sich demgegenüber im wesentlichen durch das Vorliegen einer $-CF_2-CFCl_2$-Seitenkette anstatt eines $-CF_3$-Restes in 5-Stellung am Pyridinring.

Es wurde überraschend gefunden, dass diese erfindungsgemässen Verbindungen der Formel I bei guter Pflanzenverträglichkeit und sehr geringer Warmblütertoxizität eine ausgeprägte Wirksamkeit als Schädlingsbekämpfungsmittel besitzen. Die Verbindungen eignen sich vor allem zur Bekämpfung von Pflanzen und Tiere befallenden Schädlingen. Die erfindungsgemässen Verbindungen zeichnen sich ferner durch gute Abbaubarkeit, insbesondere im Freiland, aus.

Insbesondere eignen sich die Verbindungen der Formel I zur Bekämpfung von Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera sowie von Vertretern der Ordnung Akarina.

Die gute pestizide Wirkung der erfindungsgemässen Verbindungen der Formel I entspricht einer Abtötungsrate (Mortalität) von mindestens 50-60 % der erwähnten Schädlinge.

Neben ihrer sehr günstigen Wirkung gegenüber Fliegen, wie z.B. Musca domestica, und Mückenlarven eignen sich Verbindungen der Formel I vor allem zur Bekämpfung von pflanzenschädigenden Frassinsekten, in Zier- und Nutzpflanzungen, insbesondere in Baumwollkulturen (z.B. Spodoptera littoralis und Heliothis virescens) sowie in Gemüsekulturen (z.B. Leptinotarsa decemlineata und Pieris brassicae). Hervorzuheben ist besonders die larvizide und ovizide Wirkung von Verbindungen der Formel I. Werden Verbindungen der Formel I von adulten Insekten mit dem Futter aufgenommen, so ist in vielen Fällen, insbesondere bei Coleopteren, wie z.B. Anthonomus grandis, eine verminderte Ei-Ablage und/oder reduzierte Schlupfrate festzustellen.

Die Verbindungen der Formel I eignen sich weiterhin zur Bekämpfung von Ektoparasiten, z.B. Lucilia sericata, sowie von Zecken an Haus-und Nutztieren, z.B. durch Tier-, Stall- und Weidebehandlung.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff der Formel I, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie

N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind als Tenside auch die Fettsäuremethyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual"
MC Publishing Corp., Ridgewood, New Jersey, 1979;
Dr. Helmut Stache "Tensid Taschenbuch",
Carl Hanser Verlag München/Wien 1981.

Die pestiziden Zubereitungen enthalten in der Regel 0,1 bis 99 %, insbesondere 0,1 bis 95 %, Wirkstoff der Formel I oder Kombinationen dieser Wirkstoffe mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effeke enthalten.

Beispiel 1:

a) Herstellung von 4-[5'-(2,2-Dichlor-1,1,2-trifluoräthyl-3'-chlorpyridyl-2'-oxy]-3-carbmethoxy-anilin:

16,7 g 5-Aminosalicylsäuremethylester werden in 160 ml Dimethylsulfoxid gelöst. Zu dieser Lösung werden 25 g $K_2CO_3$ zugegeben, und das Gemisch wird unter Rühren auf 90°C erwärmt. Dann werden 28,2 g 2-Fluor-3-Chlor-5-(2,2-dichlor-1,1,2-trifluoräthyl)-pyridin zugetropft, und das Gemisch wird während 5 Stunden bei 90°C gerührt. Von dem Reaktionsgemisch wird das Dimethylsulfoxid abdestilliert, der Rückstand wird mit Wasser verrührt, das Rohprodukt von der Nutsche mit Aether gelöst, die Lösung mit Wasser gewaschen und mit $Na_2SO_4$ getrocknet und der Aether abdestilliert. Das als Rückstand verbliebene Rohprodukt wird chromatographisch gereinigt (Chromatographie-Kolonne: 100 cm lang, Durchmesser 7 cm; Füllung: 1 kg Kieselgel; Lösungsmittel: Hexan 45 %, Aether 45 %, Isopropanol 10 %; Fraktionen à 150 ml). In den Fraktionen 11 - 17 ist das gereinigte Produkt enthalten. Auf diese Weise wird die Titelverbindung der Formel

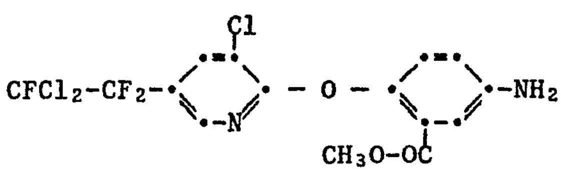

in Form beige-farbener Kristalle vom Schmelzpunkt 130-132°C erhalten.

In analoger Weise hergestellt wird das 4-[5'-(2,2-Dichlor-1,1,2-trifluoräthyl)-3'-chlorpyridyl-2-oxy]-3-carbäthoxy-5-chlor-anilin (weisse Kristalle, Smp.: 169-170°C).

b) Herstellung von N-(2,6-Difluorbenzoyl)-N'-[4-{5'-(2,2-dichlor-1,1,2-trifluoräthyl)-3'-chlorpyridyl-2'-oxy}-3-carbmethoxy-phenyl]-harnstoff.

Es werden 5,62 g des gemäss a) hergestellten 4-[5'-(2,2-Dichlor-1,1,2-trifluoräthyl)-3'-chlorpyridyl-2'-oxy]-3-carbmethoxyanilins in einer Mischung aus 50 ml Toluol und 20 ml Chloroform gelöst. Zu dieser Lösung werden 0,02 g Triäthylamin und danach noch 2,39 g 2,6-Difluorbenzoylisocyanat zugegeben. Das Reaktionsgemisch wird dann während 10 Stunden bei Raumtemperatur gerührt. Das abgeschiedene Produkt wird abgenutscht, auf der Nutsche mit Hexan gewaschen und getrocknet. Es werden farblose Kristalle der Titelverbindung der Formel

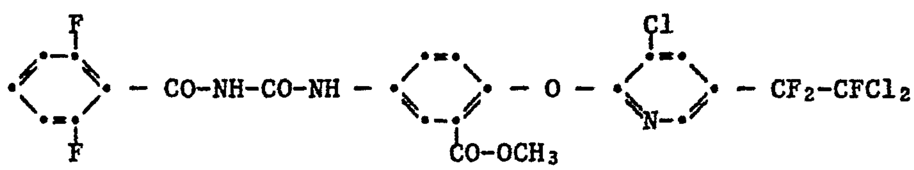

mit einem Schmelzpunkt von 215-217°C (Verbindung Nr. 1) erhalten.

Wie vorstehend beschrieben werden die folgenden Verbindungen der Formel I hergestellt:

| Verb. Nr. | $X_1$ | $X_2$ | $X_3$ | Y | R | $R_1$ | $R_2$ | $R_3$ | $R_4$ | $R_5$ | Smp. [°C] |
|---|---|---|---|---|---|---|---|---|---|---|---|
| 2 | H | H | H | Cl | $-CH_3$ | Cl | H | H | H | H | 170-171 |
| 3 | H | H | H | Cl | $-CH_3$ | $-OCH_3$ | H | H | H | H | 161-164 |
| 4 | Cl | H | H | Cl | $-CH_3$ | F | 6-F | H | H | H | 219-220 |
| 5 | Cl | H | H | Cl | $-CH_3$ | Cl | H | H | H | H | 184-185 |
| 6 | Cl | H | H | Cl | $-CH_3$ | Cl | 6-Cl | H | H | H | 133-136 |
| 7 | Cl | H | H | Cl | $-CH_3$ | F | H | H | H | H | 179-180 |
| 8 | H | H | H | Cl | H | F | 6-F | H | H | H | 202-203 |

In entsprechender Weise wie vorstehend angegeben sind auch die folgenden Verbindungen der Formel I herstellbar:

| Verb. Nr. | $X_1$ | $X_2$ | $X_3$ | Y | R | $R_1$ | $R_2$ | $R_3$ | $R_4$ | $R_5$ |
|---|---|---|---|---|---|---|---|---|---|---|
| 9 | H | H | H | Cl | $-C_2H_5$ | F | 6-F | H | H | H |
| 10 | H | H | H | Cl | $-C_3H_7(i)$ | F | 6-F | H | H | H |
| 11 | H | H | H | Cl | $-C_4H_9(n)$ | F | 6-F | H | H | H |
| 12 | Cl | H | H | H | $-CH_3$ | F | 6-F | H | H | H |
| 13 | H | H | H | Cl | $-C_4H_9(t)$ | F | 6-F | H | H | H |

Beispiel 2:

Formulierungen für Wirkstoffe der Formel I gemäss Beispiel 1 resp. Kombinationen dieser Wirkstoffe mit andern Insektiziden oder Akariziden (% = Gewichtsprozent):

| 1. Spritzpulver | a) | b) | c) |
|---|---|---|---|
| Wirkstoff oder Wirkstoff-kombination | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen vermischt und in einer geeigneten Mühle gut vermahlen.

Man erhält Spritzpulver, die sich mit Wasser zu Suspensionen jeder gewünschten Konzentration verdünnen lassen.

2. Emulsions-Konzentrat

| | |
|---|---|
| Wirkstoff oder Wirkstoffkombination | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 3. Stäubemittel | a) | b) |
|---|---|---|
| Wirkstoff oder Wirkstoffkombination | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

4. Extruder-Granulat

| | |
|---|---|
| Wirkstoff oder Wirkstoffkombination | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert, granuliert und anschliessend im Luftstrom getrocknet.

5. Umhüllungs-Granulat

| | |
|---|---|
| Wirkstoff oder Wirkstoffkombination | 3 % |
| Polyäthylenglykol (MG 200) | 3 % |
| Kaolin | 94 % |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

6. Suspensions-Konzentrat

| | |
|---|---|
| Wirkstoff oder Wirkstoffkombination | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 3: Wirkung gegen Musca domestica:
Je 50 g frisch zubereitetes Nährsubstrat für Maden werden in Becher eingewogen. Von einer 1 Gew.-%igen acetonischen Lösung des betreffenden Wirkstoffes wird eine bestimmte Menge auf das in den Bechern befindliche Nährsubstrat pipettiert, so dass sich eine Wirkstoffkonzentration von 800 ppm ergibt. Nach dem Durchmischen des Substrates lässt man das Aceton mindestens 20 Stunden lang verdampfen.

Dann werden pro Wirkstoff und Konzentration je 25 eintägige Maden von Musca domestica in die das so behandelte Nährsubstrat enthaltenden Becher gegeben. Nachdem sich die Maden verpuppt haben, werden die gebildeten Puppen durch Ausschwemmen mit Wasser von dem Substrat abgetrennt und in mit Siebdeckeln verschlossenen Gefässen deponiert. Die pro Ansatz ausgeschwemmten Puppen werden gezählt (toxischer

Einfluss des Wirkstoffes auf die Madenentwicklung). Dann wird nach 10 Tagen die Anzahl der aus den Puppen geschlüpften Fliegen bestimmt.

Verbindungen der Formel I gemäss Beispiel 1 zeigen gute Wirkung im obigen Test.

Beispiel 4: Wirkung gegen Lucilia sericata:
Zu 9 ml eines Zuchtmediums wird bei 50°C 1 ml einer 0,5 Gew.-% Aktivsubstanz enthaltenden wässrigen Zubereitung hinzugefügt. Nun werden ca. 30 frisch geschlüpfte Lucilia sericata-Larven zum Zuchtmedium gegeben, und nach 48 und 96 Stunden wird die insektizide Wirkung durch Ermittlung der Abtötungsrate festgestellt.

Verbindungen der Formel I gemäss Beispiel 1 zeigen in diesem Test gute Wirkung gegen Lucilia sericata.

Beispiel 5: Wirkung gegen Aëdes aegypti:
Auf die Oberfläche von 150 ml Wasser, das sich in einem Behälter befindet, wird so viel einer 0,1 Gew.-%igen acetonischen Lösung des Wirkstoffes pipettiert, dass eine Konzentration von 800 ppm erhalten wird. Nach Verdunsten des Acetons wird der Behälter mit 30 bis 40 2-tägigen Aëdes-Larven beschickt. Nach 1, 2 und 5 Tagen wird die Mortalität geprüft.

Verbindungen der Formel I gemäss Beispiel 1 zeigen in diesem Test gute Wirkung gegen Aëdes aegypti.

Beispiel 6: Insektizide Frassgift-Wirkung:
Ca. 25 cm hohe eingetopfte Baumwollpflanzen werden mit wässrigen Wirkstoffemulsionen besprüht, die den Wirkstoff jeweils in steigenden Konzentrationen enthalten.

Nach dem Antrocknen des Sprühbelages werden die Baumwollpflanzen mit Spodoptera littoralis- bzw. Heliothis virescens-Larven im dritten larvalen Stadium besiedelt. Der Versuch wird bei 24°C und 60 % relativer Luftfeuchtigkeit durchgeführt. Nach 120 Stunden wird die %-Mortalität der Test-Insekten bestimmt.

Die nachstehende Tabelle zeigt Ergebnisse biologischer Prüfungen erfindungsgemässer Verbindungen auf der Basis des biologischen Beispiels 6. Die Auswertung der Versuche erfolgt anhand der erhaltenen %-Mortalität unter Verwendung des folgenden Bewertungs-Index:

A: 100 % Mortalität bei einer Konzentration von 0,75 ppm der geprüften Verbindung;
B: 80-100 % Mortalität bei einer Konzentration von 3,0 ppm der geprüften Verbindung;
C: 80-100 % Mortalität bei einer Konzentration von 12,5 ppm der geprüften Verbindung;
D: 80-100 % Mortalität bei einer Konzentration von 50 ppm der geprüften Verbindung;
E: 80-100 % Mortalität bei einer Konzentration von 100 ppm der geprüften Verbindung;
F: 80-100 % Mortalität bei einer Konzentration von 200 ppm der geprüften Verbindung,
-: nicht geprüft.

| Verb. Nr. | pestizide Wirksamkeit | |
|---|---|---|
| | Spodoptera-Larven | Heliothis-Larven |
| 1 | A | C |
| 2 | B | B |
| 3 | E | E |
| 4 | B | C |
| 5 | D | C |
| 6 | F | - |
| 7 | A | C |

Beispiel 7: Ovizide Wirkung auf Heliothis virescens:
Entsprechende Mengenanteile einer benetzbaren pulverförmigen Formulierung, enthaltend 25 Gew.% des zu prüfenden Wirkstoffes, werden mit jeweils soviel Wasser vermischt, dass sich eine wässrige Emulsion mit einer Wirkstoffkonzentration von 800 ppm ergibt.

In diese wirkstoffhaltigen Emulsionen werden eintägige Eigelege von Heliothis auf Cellophan während drei Minuten eingetaucht und dann auf Rundfilter abgenutscht. Die so behandelten Gelege werden in Petrischalen ausgelegt und in der Dunkelheit aufbewahrt. Nach 6 bis 8 Tagen wird die Schlupfrate im Vergleich zu unbehandelten Kontrollen festgestellt.

Verbindungen der Formel I gemäss Beispiel 1 zeigen gute Wirkung in obigem Test.

Beispiel 8: Wirkung auf Laspeyresia pomonella (Eier):
Abgelegte Eier von Laspeyresia pomonella, die nicht älter als 24 Stunden sind, werden auf Filterpapier für 1 Minute in eine acetonisch-wässrige Lösung, enthaltend 800 ppm des zu prüfenden Wirkstoffes, eingetaucht. Nach dem Antrocknen der Lösung werden die

Eier in Petrischalen ausgelegt und bei einer Temperatur von 28°C belassen. Nach 6 Tagen wird der prozentuale Schlupf aus den behandelten Eiern bewertet und die %-Mortalität bestimmt.

Verbindungen der Formel I gemäss Beispiel 1 zeigen gute Wirkung in obigem Test.

Beispiel 9: Reproduktions-Beeinflussung von Anthonomus grandis:
Adulte Anthonomus grandis, die nach dem Schlupf nicht älter als 24 Stunden waren, werden in Gruppen zu jeweils 25 Käfern in Käfige mit Gitterwänden überführt. Die mit den Käfern besetzten Käfige werden sodann während 5 bis 10 Sekunden in eine acetonische Lösung, enthaltend 0,1 Gew.% des zu prüfenden Wirkstoffes, eingetaucht. Nachdem die Käfer wieder trocken sind, werden sie zur Kopulation und Eiablage in abgedeckte und Futter enthaltende Schalen eingesetzt. Abgelegte Eier werden zwei- bis dreimal wöchentlich mit fliessendem Wasser ausgeschwemmt, gezählt, durch zwei- bis dreistündiges Einlegen in ein wässriges Desinfektionsmittel desinfiziert und dann in Schalen, die eine geeignete Larvaldiät enthalten, deponiert. Nach 7 Tagen wird untersucht, ob sich aus den deponierten Eiern Larven entwickelt haben.

Zur Ermittlung der Dauer des die Reproduktion beeinflussenden Effektes der zu prüfenden Wirkstoffe wird die Eiablage der Käfer während eines Zeitraums von etwa 4 Wochen überprüft. Die Bonitierung erfolgt anhand der Verminderung der Anzahl abgelegter Eier und der daraus geschlüpften Larven im Vergleich zu unbehandelten Kontrollen.

Verbindungen der Formel I gemäss Beispiel 1 zeigen eine gute reproduktionshemmende Wirkung im obigen Test.

Beispiel 10: Wirkung gegen Anthonomus grandis (Adulte)
Zwei eingetopfte Baumwollpflanzen im 6-Blattstadium werden jeweils mit einer wässrigen benetzungsfähigen Emulsions-Zubereitung enthaltend 400 ppm des zu prüfenden Wirkstoffes besprüht. Nach dem Antrocknen des Spritzbelages (nach etwa 1,5 Stunden) wird jede

Pflanze mit 10 adulten Käfern (Anthonomus grandis) besiedelt. Plastikzylinder, deren obere Oeffnungen mit Gaze abgedeckt sind, werden dann über die behandelten, mit den Testtieren besiedelten Pflanzen gestülpt, um ein Abwandern der Käfer zu verhindern. Die so behandelten Pflanzen werden bei 25°C und etwa 60 % relativer Luftfeuchtigkeit gehalten. Die Auswertung erfolgt nach 2, 3, 4 und 5 Tagen unter Zugrundelegung der prozentualen Mortalität der eingesetzten Testtiere (% Rückenlage) sowie der Antifeedant-Wirkung gegenüber unbehandelten Kontrollansätzen.

Verbindungen der Formel I gemäss Beispiel 1 zeigen gute Wirkung in obigem Test.

Patentansprüche

1. Verbindung der Formel

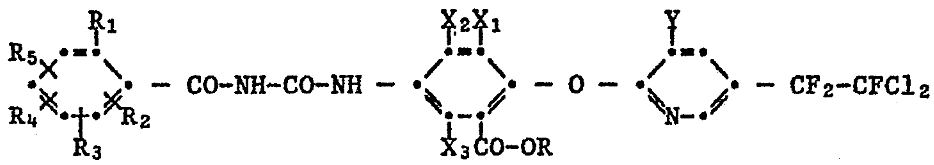 (I),

worin

$X_1$, $X_2$ und $X_3$ unabhängig voneinander Wasserstoff, Halogen oder Methyl;

Y Wasserstoff oder Halogen;

R Wasserstoff, $C_1$-$C_5$-Alkyl, $C_3$-$C_5$-Alkenyl oder $C_3$-$C_5$-Alkinyl; und

$R_1$, $R_2$, $R_3$, $R_4$ und $R_5$ unabhängig voneinander Wasserstoff, Halogen, $C_1$-$C_4$-Alkyl, $C_1$-$C_4$-Alkoxy oder $C_1$-$C_4$-Alkylthio

bedeuten.

2. Verbindung der Formel I gemäss Anspruch 1, dadurch gekennzeichnet, dass

$X_1$, $X_2$ und $X_3$ unabhängig voneinander Wasserstoff, Fluor, Chlor oder Methyl;

Y Wasserstoff, Fluor oder Chlor;

R Wasserstoff, $C_1$-$C_4$-Alkyl, Allyl oder Propargyl; und

$R_1$, $R_2$, $R_3$, $R_4$ und $R_5$ unabhänig voneinander Wasserstoff, Fluor, Chlor, Methyl, Aethyl, Methoxy, Aethoxy oder Methylthio

bedeuten.

3. Verbindung der Formel I gemäss Anspruch 2, dadurch gekennzeichnet, dass

$X_1$ Wasserstoff, Fluor, Chlor oder Methyl;

$X_2$ und $X_3$ Wasserstoff;

Y Wasserstoff oder Chlor;

R Wasserstoff oder $C_1$-$C_4$-Alkyl;

$R_1$ Fluor, Chlor, Methyl, Aethyl, Methoxy, Aethoxy oder Methylthio;

$R_2$ Wasserstoff, 6-Fluor, 6-Chlor, 6-Methyl oder 6-Aethyl; und

$R_3$, $R_4$ und $R_5$ Wasserstoff
bedeuten.

4. Verbindung der Formel I gemäss Anspruch 3, dadurch gekennzeichnet, dass
$X_1$ Wasserstoff oder Chlor;
$X_2$ und $X_3$ Wasserstoff;
Y Wasserstoff oder Chlor;
R Wasserstoff oder $C_1$-$C_4$-Alkyl;
$R_1$ Fluor, Chlor oder Methoxy;
$R_2$ Wasserstoff, 6-Fluor oder 6-Chlor; und
$R_3$, $R_4$ und $R_5$ Wasserstoff
bedeuten.

5. Verbindung gemäss Anspruch 4 der Formel

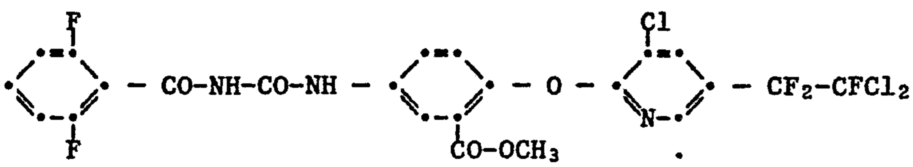

6. Verbindung gemäss Anspruch 4 der Formel

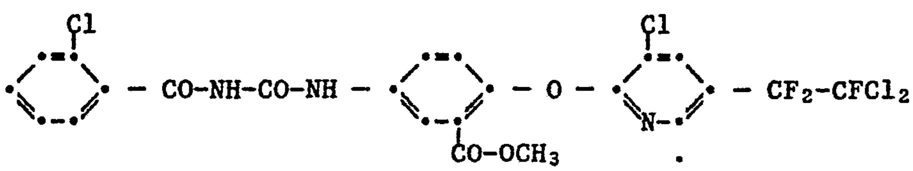

7. Verbindung gemäss Anspruch 4 der Formel

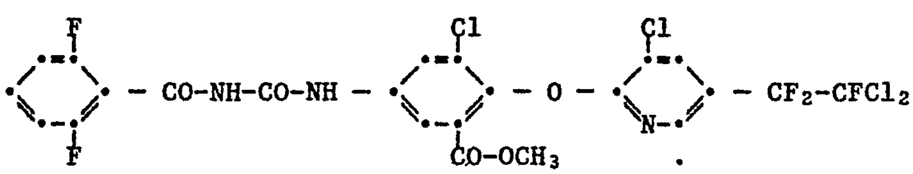

8. Verbindung gemäss Anspruch 4 der Formel

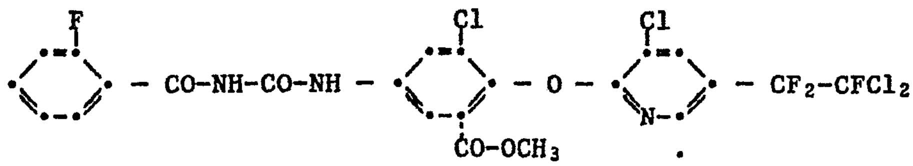

9. Verfahren zur Herstellung einer Verbindung der Formel I gemäss einem der Ansprüche 1 bis 8, dadurch gekennzeichnet, dass man

a) eine Verbindung der Formel II

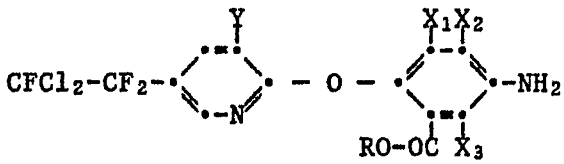

(II)

mit einer Verbindung der Formel III

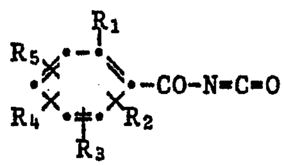

(III),

b) eine Verbindung der Formel IV

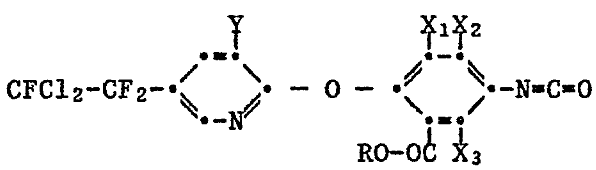

(IV)

mit einer Verbindung der Formel V

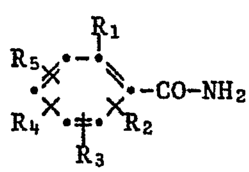

(V) oder

c) eine Verbindung der Formel II mit einer Verbindung der Formel VI

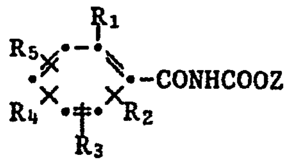

(VI)

umsetzt, wobei in den Formeln II bis VI die Reste $X_1$ bis $X_3$, Y, R und $R_1$ bis $R_5$ die in den Ansprüchen 1 bis 4 angegebenen Bedeutungen haben, und Z eine gegebenenfalls mit Halogen substituierte $C_1$-$C_8$-Alkylgruppe bedeutet.

10. Verbindung der Formel II

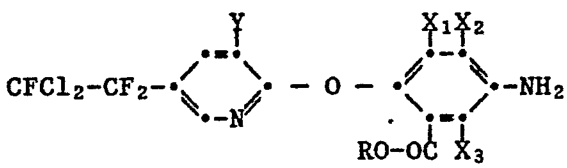

(II)

worin

$X_1$ , $X_1$ und $X_3$ unabhängig voneinander Wasserstoff, Halogen oder Methyl;

Y Wasserstoff oder Halogen; und

R Wasserstoff, $C_1$-$C_5$-Alkyl, $C_3$-$C_5$-Alkenyl oder $C_3$-$C_5$-Alkinyl bedeuten.

11. Schädlingsbekämpfungsmittel, welches als aktive Komponente eine Verbindung gemäss einem der Ansprüche 1 bis 8 zusammen mit geeigneten Trägern und/oder anderen Zuschlagstoffen enthält.

12. Verwendung einer Verbindung gemäss einem der Ansprüche 1 bis 8 zur Bekämpfung von Insekten und Vertretern der Ordnung Akarina.

13. Verwendung gemäss Anspruch 12 zur Bekämpfung larvaler Stadien pflanzenschädigender Insekten.

14. Verfahren zur Bekämpfung von Insekten und Vertretern der Ordnung Akarina, dadurch gekennzeichnet, dass man die Insekten bzw. deren verschiedene Entwicklungsstadien oder deren Aufenthaltsort mit einer

pestizid wirksamen Menge einer Verbindung der Formel I gemäss einem der Ansprüche 1 bis 8 oder mit einem Mittel enthaltend neben Zusatz-und Trägerstoffen eine pestizid wirksame Menge dieser Verbindung, in Kontakt bringt oder behandelt.

FO 7.5 EIC/eg*